# FASCICULE DE BREVET EUROPEEN

(11) **EP 1 857 835 B1**
(45) Date de publication et mention de la délivrance du brevet: **24.12.2008**
(21) Numéro de dépôt: 07108105.3
(22) Date de dépôt: 14.05.2007
(51) Int. Cl.: G01T 1/16, A61N 5/10

(54) **Système de maintien et de positionnement de sources radioactives miniatures par levitation aerodynamique et application à un dispositif de caractérisation en continu d'implants prostatiques**
Halte- und Positionierungssystem von radioaktiven Miniquellen durch aerodynamisches Schwebesysem und Anwendung auf eine Vorrichtung zur kontinuierlichen Charakterisierung von Prostataimplantaten
System for holding and positioning miniature radiation sources by aerodynamic levitation and application to a device for continuous characterisation of prostate implants

(30) Priorité: 16.05.2006 FR 0651757
(43) Date de publication de la demande: 21.11.2007
(73) Titulaire: COMMISSARIAT A L'ENERGIE ATOMIQUE, 75015 Paris (FR)
(72) Inventeur: Douysset, Guilhem, 78220 Viroflay (FR)
(74) Mandataire: Poulin, Gérard

(56) Documents cités:
- EP-A1- 0 337 908
- US-A- 4 378 209
- US-B1- 6 168 638
- CULBERSON W ET AL: "Large-volume ionization chamber with variable apertures for air-kerma measurements of low-energy radiation sources" REVIEW OF SCIENTIFIC INSTRUMENTS, AMERICAN INSTITUTE OF PHYSICS, US, vol. 77, no. 1, 19 janvier 2006 (2006-01-19), pages 15105-15105, XP012092326 ISSN: 0034-6748

## Description

### DOMAINE TECHNIQUE

L'invention proposée concerne système de maintien et de positionnement d'au moins un élément miniaturisé se présentant sous la forme d'un cylindre et en particulier de sources radioactives miniatures dans le but de les caractériser. Ce dispositif peut être utilisé comme élément central d'un détecteur multi-capteurs permettant de caractériser en continu et de manière complète des implants radioactifs destinés, entre autres, à la curiethérapie des cancers de la prostate. Cependant, bien que l'invention ait été conçue en vue de caractériser des sources radioactives, elle peut être employée à d'autres fins, pour mettre un petit cylindre d'un matériau quelconque en lévitation.

### ÉTAT DE LA TECHNIQUE ANTÉRIEURE

La curiethérapie représente une alternative intéressante à la chirurgie ou à la radiothérapie conventionnelle pour le traitement de certains cancers. C'est notamment le cas pour le traitement des cancers de la prostate. De nos jours, ce type de pathologie connaît une forte recrudescence en partie du fait du dépistage précoce.

La curiethérapie par implants permanents consiste à irradier la tumeur en plaçant directement à l'intérieur de la prostate du malade une centaine de sources miniatures d'iode-125 ou de palladium-103 (le premier isotope étant de loin le plus utilisé). L'implantation est réalisée en une seule fois sous anesthésie locale. Le protocole est de ce fait plus facile à tolérer pour le patient (une trentaine de séances sont souvent nécessaires en irradiation externe). Par rapport à l'irradiation externe cette approche permet de plus de limiter la dose délivrée aux tissus sains environnants tout en maximisant la dose délivrée à la tumeur. Les isotopes sont en effet sélectionnés pour la faible énergie de leur spectre d'émission (de 15 keV à 35 keV) et donc la faible pénétration de leur rayonnement.

La décroissance radioactive est également rapide (la période radioactive de l'iode-125 est de 59 jours et celle du palladium-103 est de 17 jours) : quelques mois après l'implantation le corps du patient ne contient virtuellement plus de matière radioactive, les sources ne sont donc pas retirées.

Ce mode de traitement, pratiqué depuis une dizaine d'années aux USA, a montré que les résultats obtenus en terme de contrôle tumoral étaient comparables aux autres techniques de traitement (chirurgie, radiothérapie externe). Le véritable apport se situe au niveau des effets secondaires. Il est en effet actuellement admis que l'occurrence des complications post-traitement était significativement plus faible avec cette technique. Toutefois, l'expérience montre que ce bénéfice est obtenu pleinement pour les cancers de stades initiaux. Cependant grâce au dépistage systématique actuellement mis en place, une grande proportion des cancers diagnostiqués le sont précisément dans ces stades initiaux et chez des personnes jeunes pour lesquelles l'absence d'effets secondaires participe largement au choix thérapeutique.

Actuellement, pour ne prendre en compte que la situation américaine, près de 230 000 cancers de la prostate sont diagnostiqués par an. Environ 100 000 traitements par implants sont effectués annuellement. La curiethérapie représente donc un marché important et en quelques années près d'une vingtaine d'industriels ont entrepris la fabrication de sources. En Europe, et en particulier en France, ce mode de traitement est encore en phase de démarrage, on estime qu'il devrait néanmoins connaître un essor rapide ces prochaines années.

Les sources employées présentent les caractéristiques principales suivantes :
- Faible activité radioactive individuelle (de 3 à 600 MBq soit un débit de dose de 0,1 à 20 µGy/h à 1 m pour l'isotope iode-125). Ceci rend relativement délicates les mesures dosimétriques (faible rapport signal/bruit)
- Faibles dimensions. Plusieurs dizaines de types de sources différentes existent sur le marché, toutefois elles sont toutes de formes cylindriques (diamètre compris entre 0,7 et 0,8 mm, hauteur comprise entre 4,5 et 5,5 mm). Leur masse est de l'ordre de la dizaine de milligrammes. Leur préhension et leur manipulation sont relativement délicates. Etant donnée la faible épaisseur du gainage (de l'ordre de 50 µm de titane) les sources sont relativement fragiles.
- Faible énergie des rayonnements émis. Ceci est nécessaire pour confiner la dose délivrée à la seule région tumorale. Toutefois, cela rend sensible le spectre d'émission à l'environnement : absorption des photons dans la gaine, dans l'air, diffusion des photons par les matériaux environnants... Ceci conduit notamment à une très forte anisotropie angulaire de l'émission (jusqu'à ± 5% de variation de dose pour une rotation de 360° autour de l'axe principal de la source). De plus, les sources contiennent parfois des marqueurs à base de métaux denses (Ag, Au) pour faciliter leur localisation en radiographie. La fluorescence induite par ces matériaux modifie profondément le spectre émis et par conséquent la dose délivrée.

Comme toujours en radiothérapie, il est essentiel de connaître très précisément la dose délivrée au patient : quelques pourcents de surdosage ou de sous-dosage conduisent systématiquement à des complications ou des récidives. Avant l'implantation, il est donc nécessaire pour le thérapeute et le physicien médical de disposer d'un moyen de mesure des caractéristiques dosimétriques des sources qui vont être implantées. Comme le nombre de sources utilisées est important et que la mesure est relativement délicate à réaliser, il est fréquent que seule une partie du lot de sources soit mesurée. Pour cela, les services hospitaliers disposent généralement de chambres d'ionisation à puits (détecteur de forme cylindrique dans lequel la source est totalement insérée, voir le document [1] cité en fin de description). Ces instruments sont de conception assez simple mais ils requièrent un ré-étalonnage périodique par les services de métrologie habilités.

En revanche, pour réaliser des mesures absolues, les caractéristiques particulières des sources obligent de disposer d'une instrumentation spécifique. Cette instrumentation est développée uniquement au niveau des laboratoires nationaux de métrologie. Une partie importante de la difficulté réside dans la réalisation du dispositif de maintien de la source face au détecteur. Il faut réaliser le compromis délicat suivant : la source doit être maintenue fermement sans pour autant utiliser de matériaux perturbant le spectre d'émission. La source doit pouvoir être placée de manière reproductible face au détecteur et il faut de plus disposer d'un moyen de réaliser une rotation de la source au cours de la mesure afin de compenser l'anisotropie de l'émission.

A ce jour, les laboratoires nationaux de métrologie ont été les principaux acteurs dans le domaine. Aux Etats-Unis, le NIST (National Institute of Standards and Technology) a développé depuis plusieurs années un détecteur capable de réaliser des mesures absolues des doses délivrées par ces sources. Le dispositif de maintien de la source est relativement basique : la source est maintenue à la verticale par un adhésif lui même adapté à un moteur permettant de réaliser la rotation (document [2]). Toujours aux Etats-Unis, le laboratoire de dosimétrie accrédité de l'université du Wisconsin a récemment également développé un instrument. La source est maintenue en position face au détecteur à l'aide d'un système de quatre fils de nylon tendus au milieu desquels la source est insérée. La rotation est assurée par les deux moteurs auxquels les fils sont fixés (document [3]). En Allemagne, le PTB (Physikalish-Technische Bundesanstalt) a récemment mis au point un détecteur en collaboration avec le laboratoire précédent. Le dispositif support de source est de conception identique à celui mis en oeuvre au NIST.

Ces trois systèmes présentent de nombreux inconvénients. Le premier est la difficulté d'utilisation: la source doit être positionnée précisément dans le dispositif à l'aide d'outils de préhension (les sources sont trop actives pour être manipulées à la main). Le risque de mauvais positionnement, voire de perte de la source, est important. Le second inconvénient est le caractère relativement invasif des dispositifs : le spectre d'émission des sources peut être perturbé significativement par le système de maintien. Enfin l'inconvénient, sans doute majeur de ces systèmes, est leur limitation en terme de productivité : les sources doivent être introduites manuellement une par une dans les dispositifs. Ceci empêche de les utiliser pour mesurer rapidement un grand nombre de sources.

Les détecteurs utilisés au niveau des services hospitaliers (chambres d'ionisation à puits), ne sont pas non plus adaptés pour traiter un grand nombre de sources. La source faisant l'objet d'une mesure doit être introduite manuellement dans un adaptateur (voir par exemple le document [4]) situé dans la partie centrale du détecteur. Puis, après la mesure, la source doit être stérilisée avant l'implantation. De ce fait, la plupart des centres ne mesurent qu'une partie du lot destiné à être implanté.

Afin d'augmenter la productivité des détecteurs, le document [5] divulgue un système automatique de tri d'implants en fonction de leurs caractéristiques dosimétriques. Les sources sont transportées individuellement à l'horizontale par un système purement mécanique depuis un « réservoir » à travers une chambre d'ionisation où les débits de dose sont mesurés et finalement vers un système de tri (godets). Selon les auteurs, quelques milliers de sources peuvent être mesurées automatiquement une à une et réparties en différents lots. Ce dispositif est probablement satisfaisant pour la mesure dosimétrique, en revanche l'utilisation massive d'éléments mécaniques perturbe fortement les spectres d'émission. Ce système ne peut donc fonctionner correctement qu'après un étalonnage avec une source connue de configuration identique à celles qui devront être triées. Il ne peut, en revanche, en aucun cas être utilisé pour des caractérisations spectroscopiques. Les nombreux systèmes mécaniques doivent également être contrôlés de manière précise pour éviter toute destruction accidentelle des sources.

Des systèmes plus généraux de maintien sans contact de petits objets existent. Le document [6] présente par exemple un dispositif de préhension sans contact et de transport d'objets sphériques miniatures. Le système proposé fonctionne sur le principe de la lévitation aérodynamique. L'objet est maintenu par un flux de gaz à quelques millimètres au-dessus d'une pièce d'injection de gaz en forme de tuyère divergente. Ce dispositif s'adresse uniquement à des objets de formes sphériques avec comme champ d'application principal la micro-électronique. Contrairement au cas où l'objet est de forme cylindrique, le mouvement créé ici est stable, ceci simplifie notablement la configuration de l'appareil. L'objet peut être déplacé verticalement sur quelques millimètres mais le système n'est pas conçu pour cette fonction.

Le document US-A-4 378 209 présente également un système de maintien et de positionnement d'au moins un élément miniaturisé selon le préambule de la revendication 1.

### EXPOSE DE L'INVENTION

La présente invention a pour but de remédier aux inconvénients présentés par les systèmes de l'art antérieur.

Elle a pour objet un système de maintien et de positionnement d'au moins un élément miniaturisé se présentant sous la forme d'un cylindre, le système comprenant un tube avec une partie supérieure, une partie médiane et une partie inférieure, la partie supérieure étant pourvue d'un orifice d'introduction de l'élément et permettant le déplacement de l'élément jusqu'à la partie médiane qui présente un diamètre légèrement supérieur au diamètre du cylindre formant l'élément, la partie inférieure étant raccordée à des moyens d'injection contrôlée d'un gaz pour injecter un gaz à l'intérieur du tube de manière à mettre l'élément en lévitation à une hauteur désirée dans la partie médiane du tube, la partie inférieure du tube étant pourvue d'un orifice d'évacuation de l'élément, le tube comprenant également des moyens d'élimination de l'électricité statique présente à l'intérieur du tube.

Avantageusement, la partie supérieure du tube est d'un diamètre supérieur au diamètre de la partie médiane du tube et est reliée à la partie médiane par une partie en forme d'entonnoir.

Avantageusement, le diamètre de la partie médiane du tube est croissant en partant de la partie inférieure du tube jusqu'à arriver à la partie supérieure du tube.

Les moyens d'injection d'un gaz peuvent être des moyens d'injection d'un gaz choisi parmi les gaz rares, l'air, l'azote et un mélange d'azote et d'oxygène. Le gaz peut être injecté grâce à un débitmètre, de préférence grâce à un débitmètre massique.

Les moyens d'élimination de l'électricité statique peuvent comprendre un fil électriquement conducteur disposé à l'intérieur du tube et relié à la terre. Ils peuvent aussi comprendre des moyens d'apport à l'intérieur du tube de charges électriques opposées à celles fournies par l'électricité statique. Ces moyens d'apport de charges peuvent comprendre un fil électriquement conducteur disposé à l'intérieur du tube et porté à un potentiel électrique lui permettant de produire lesdites charges électriques.

Le système peut comprendre aussi un capteur de position de la source asservissant le débit de gaz injecté à l'intérieur du tube de façon à positionner ledit élément à la hauteur désirée.

L'invention s'applique en particulier au cas où l'élément miniaturisé est une source radioactive.

L'invention a aussi pour objet un appareil de caractérisation d'au moins une source radioactive miniaturisée, caractérisé en ce qu'il comprend un système de maintien et de positionnement tel que défini ci-dessus et des moyens de caractérisation de l'élément miniaturisé qui est une source radioactive.

Les moyens de caractérisation peuvent comprendre au moins un dispositif choisi parmi un spectromètre, une caméra vidéo, un dosimètre et un tube à rayons X et le capteur associé.

L'appareil peut comprendre des moyens pour injecter successivement les sources radioactives dans le tube, ces moyens d'injection injectant chaque nouvelle source à caractériser après que la source injectée précédemment a été mise en lévitation, caractérisée et éjectée du tube. Il peut comprendre en outre des moyens d'obturation de l'extrémité inférieure du tube pour obturer le tube pendant la mise en lévitation et la caractérisation de la source introduite dans le tube et pour ouvrir le tube afin de récupérer la source caractérisée.

### BRÈVE DESCRIPTION DU DESSIN

L'invention sera mieux comprise et d'autres avantages et particularités apparaîtront à la lecture de la description qui va suivre, donnée à titre d'exemple non limitatif, accompagnée des dessins annexés parmi lesquels :
- la figure 1 représente un schéma de principe d'un dispositif de caractérisation en continu d'implants prostatiques selon l'invention,
- la figure 2 représente une variante de tube utilisable pour la présente invention.

### EXPOSÉ DÉTAILLÉ DE MODES DE RÉALISATION PARTICULIERS

Le cahier des charges du système qui a été développé comportait les éléments suivants :
- Perturbation du spectre d'émission aussi faible que possible : utilisation d'éléments de faibles numéros atomiques uniquement (en faibles épaisseurs).
- Possibilité de positionnement précis de la source dans toutes les directions.
- Possibilité de translation verticale de la source sur une plage d'au moins 3 cm.
- Possibilité de traiter rapidement un grand nombre de sources.
- Simplicité d'utilisation, sûreté intrinsèque du dispositif (pas de risque de mauvais positionnement ou de perte de source)

Le système qui a été développé a pour principe de réaliser la lévitation de la source par un flux de gaz. Comme la source est de forme cylindrique, le seul moyen d'obtenir un effet de lévitation stable est de la placer dans un tube vertical de diamètre interne très légèrement supérieur à celui de la source.

Le système conçu reprend le principe du rotamètre mais de manière inversée. Les rotamètres sont des instruments communément employés pour mesurer les débits de fluide. Ils se composent d'un tube vertical de section variable dans lequel flotte une bille ou un pointeau. Cette bille se déplace face à une échelle graduée d'autant plus haut que le débit du fluide est important.

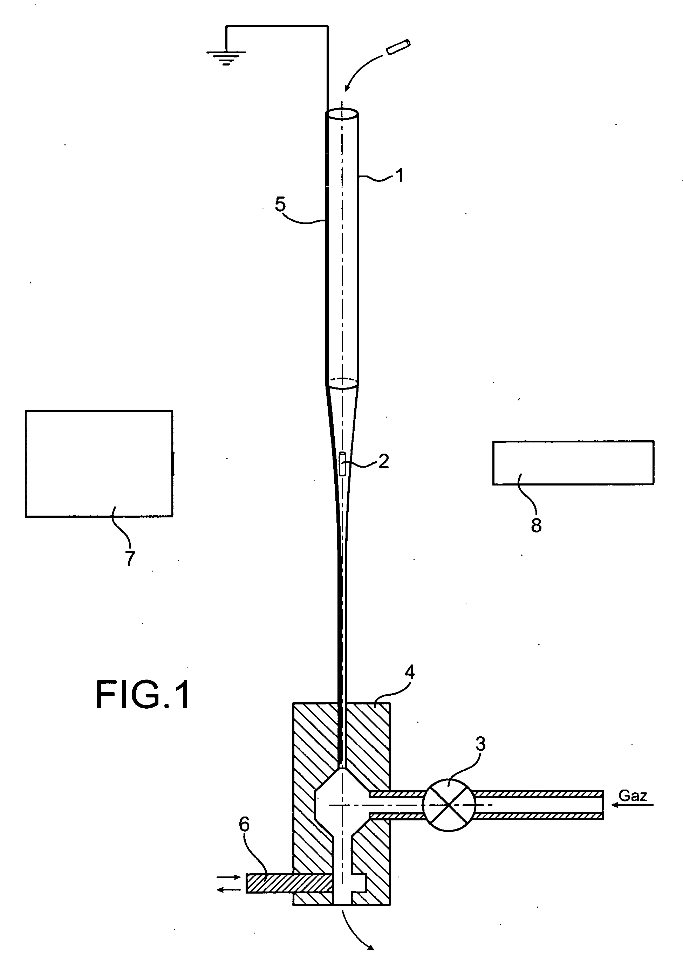

La figure 1 est un schéma de principe d'un dispositif de caractérisation en continu d'implants prostatiques selon l'invention.

Ce dispositif comprend un tube cylindrique 1 contenant la source 2. Le tube 1 est alimenté en gaz à partir d'un débitmètre massique de précision 3 injectant le gaz en partie basse du tube grâce à une pièce d'adaptation 4. Le tube 1 comprend une partie inférieure de diamètre constant, une partie médiane de diamètre croissant à mesure que l'on s'éloigne de la partie inférieure, et une partie supérieure de diamètre constant. Le changement du débit du gaz injecté permet de placer la source 2 à la hauteur voulue. Le principe est relativement simple : le poids de la source 2 est compensé par la force aérodynamique due au gaz injecté. Cette force est proportionnelle à la section de la source et au carré de la vitesse du flux l'air. Le débit de gaz est égal au produit de la vitesse de l'écoulement par la section du tube et est une grandeur conservative. Pour déplacer la source 2 vers le haut, il faut donc augmenter le débit d'air soufflé pour compenser l'augmentation de diamètre du tube. En pratique, étant donnée la faible masse des sources des débits de gaz inférieurs au litre par minute sont suffisants. Etant donnée la forme cylindrique de la source, le mouvement obtenu ne peut être stable. La source oscille à fréquence élevée de manière latérale. Toutefois, comme l'espace entre la source 2 et la paroi interne du tube 1 est limité, ceci est sans conséquence pour la mesure dosimétrique (la position moyenne demeure centrée sur l'axe du tube). En revanche, un avantage primordial du dispositif est que la source entre spontanément en rotation sur elle-même. La vitesse de cette rotation dépend du débit injecté, elle est de l'ordre de quelques tours par seconde. Ceci permet de s'affranchir de manière très simple de tous les systèmes de motorisation utilisés par les dispositifs concurrents pour compenser l'anisotropie de l'émission.

Le système présenté paraît simple à mettre en oeuvre au premier abord mais dans la réalité des difficultés apparaissent. En effet, sans précaution particulière ce système fonctionne pendant quelques minutes puis soudainement la source vient se coller à la paroi du tube. Ce collage est d'ailleurs très efficace puisqu'il est très difficile par la suite de décoller la source. Différents phénomènes sont à l'origine de ce comportement. Le premier, et le plus évident, est le collage par capillarité. Si le tube ou les sources présentent la moindre trace de graisse, ce type de collage mécanique peut survenir. La première précaution à prendre est donc de nettoyer soigneusement les éléments avant utilisation (un rinçage avec une solution d'acétone et/ou d'éther est suffisant). Le même effet peut être observé si le gaz injecté n'est pas parfaitement exempt de graisse et de vapeur d'eau. Il faut donc veiller à utiliser du gaz en bouteille (azote, azote/oxygène 80/20, gaz rare, par exemple l'argon) qui, par nature est sec et exempt de graisse. On peut également utiliser de l'air comprimé issu d'un circuit de distribution central à condition de lui adjoindre les systèmes de filtration nécessaires.

Le second phénomène expliquant les collages intempestifs est plus insidieux : il s'agit de l'électricité statique. En effet, comme la source est en collision constante avec le tube et que ce dernier comme la source sont isolés électriquement, les deux éléments se chargent peu à peu en électricité statique. Comme les sources sont de très faibles masse (10 mg environ), la force électrostatique qui apparaît, quoique de très faible amplitude, est suffisante pour les retenir contre la paroi. Il est intéressant de noter que ce phénomène n'apparaît pas avec les rotamètres usuels du fait de la masse importante (quelques grammes) et de la forme des flotteurs utilisés. L'effet est, en outre, d'autant plus marqué que la surface interne du tube est peu conductrice. Or, c'est précisément le cas puisque, pour les raisons évoquées plus haut, les tubes doivent être soigneusement nettoyés. Le problème est également amplifié par le fait que le gaz soufflé est très sec. Il n'est cependant pas envisageable de l'humidifier car les risques de collage par capillarité seraient alors importants. Pour s'affranchir de cet effet, il faut donc développer un moyen permettant de neutraliser les charges produites. Pour cela, on a inséré à l'intérieur du tube un fil métallique très fin 5 (par exemple d'un diamètre compris entre 5 µm et 15 µm) relié à la terre. Le fil 5 est plaqué contre la paroi du tube. Comme la source 2 est en constante collision avec la paroi du tube 1, elle vient périodiquement en contact avec le fil 5 et peut donc se décharger. Ce dispositif fonctionne parfaitement et, une fois mis en place, plus aucun collage n'apparaît même après plusieurs heures de fonctionnement. Le diamètre du fil 5 est très faible et n'occulte donc qu'une infime partie de la surface de la source. De plus, il peut être réalisé en matériau de faible numéro atomique (Al, Al/Si, fibre de carbone par exemple) ce qui limite dans une très grande mesure la perturbation du spectre photonique. Un autre moyen de neutraliser l'électricité statique pourrait également être mis en oeuvre : l'apport délibéré de charges électriques opposées à celles créées spontanément. Pour cela, on utilise à nouveau un fil métallique de faible diamètre mais il n'est placé qu'en deçà de la région où se trouve la source. Ce fil est porté à une tension élevée (quelques milliers de volts) et des charges sont produites à son extrémité par effet de pointe. Ces charges sont transportées sur quelques centimètres par le flux de gaz et viennent neutraliser le phénomène. Cette solution permet de réduire à zéro l'influence du fil puisque celui-ci ne se situe pas à la hauteur de la source mais elle est sans nul doute plus complexe de mise en oeuvre. En outre, la compensation des charges est plus délicate à doser que leur simple neutralisation.

En ce qui concerne le tube 1 contenant la source 2, il peut être réalisé avec n'importe quel matériau. Il sera toutefois préférable de choisir un matériau transparent (verre, plastique, quartz...) afin de pouvoir observer directement la source 2. La fabrication du tube pourra être réalisée sur le modèle de la pipette de prélèvement (ou pipette Pasteur). Ces pipettes sont fabriquées par étirement à chaud à partir d'un tube de section constante. Elles présentent donc, par nature, la section variable nécessaire au fonctionnement du dispositif. Dans la partie supérieure, elles présentent une forme évasée, le diamètre interne est alors relativement important (typiquement compris entre 6 mm et 8 mm). Cette forme facilite grandement la mise en place de la source dans le tube. En effet, il suffit d'activer le flux de gaz et de laisser tomber la source par gravité dans le tube. La forme « d'entonnoir » en partie médiane du tube conduit alors automatiquement la source jusqu'à la position d'équilibre. En outre, le diamètre supérieur du tube étant important le débitmètre massique d'injection de gaz 3 peut être choisi de sorte que le débit maximum de soufflage ne soit pas suffisant pour parvenir à éjecter la source par l'extrémité supérieure du tube. Ceci renforce notablement la sécurité du dispositif.

On veillera à optimiser l'épaisseur et la nature des parois du tube afin de minimiser l'absorption des photons et la modification du spectre d'émission. Par exemple, une paroi de quartz (SiO₂) de l'ordre de 100 µm d'épaisseur est facilement réalisable et répond à ces contraintes : transmission supérieure à 98% à 30 keV et absence d'éléments lourds pouvant conduire à des fluorescences parasites. Ce tube assure de plus la filtration des raies de fluorescence du matériau constituant la gaine de la source (du titane généralement). Cette filtration est en effet nécessaire pour réaliser des mesures dosimétriques précises. Il peut également être opportun de choisir un matériau à faible coefficient de dilatation thermique. En effet, si suite à des variations de température le diamètre interne du tube est modifié, la position d'équilibre de la source en serait affectée.

L'ensemble du dispositif présenté ici a été construit et ses performances ont été testées à l'aide de sources radioactives factices. La stabilité verticale de la source qui est un des paramètres essentiels a été mesurée : elle est actuellement meilleure que ± 0,5 mm sur plusieurs heures. Ce point pourrait sans doute être encore amélioré notamment par l'utilisation d'un système de rétro-contrôle (capteur optique de position de la source asservissant le flux de gaz injecté). Toutefois pour les applications visées les performances actuelles semblent suffisantes. La résolution du débitmètre massique d'injection de gaz 3 (typiquement 0,001 1/min) est également suffisante pour réaliser de très faibles incréments dans les déplacements verticaux (de l'ordre de ± 200 µm).

L'application immédiate de ce dispositif concerne les dispositifs de mesures dosimétriques absolues des implants prostatiques. Toutefois ce marché est symbolique : il n'intéresse potentiellement au maximum qu'une petite dizaine de laboratoires nationaux de métrologie. En revanche, le système développé présente un avantage notable par rapport aux systèmes concurrents: il est possible de l'automatiser.

Pour cela, il faut prévoir un système d'injection des sources une à une dans le tube. On peut, par exemple, réaliser l'injection de gaz de manière latérale et adjoindre une vanne pilotable 6 (ou tout autre élément assurant la même fonction) destinée à obturer l'orifice d'éjection durant la lévitation de la source. Le débitmètre massique doit également disposer d'une interface de commande (type RS232).

Un tel système peut fonctionner selon le cycle suivant :
- mise en route de l'injection de gaz par le débitmètre 3 ;
- injection automatique d'une source 2 dans la partie supérieure du tube 1 ;
- réglage du flux de gaz par le débitmètre 3 afin d'amener la source 2 en lévitation à la hauteur voulue pour la mesure ;
- mesure des caractéristiques de la source 2 en lévitation ;
- ouverture de la vanne et arrêt éventuel de l'injection de gaz, la source tombe alors par gravité dans la partie inférieure du tube 1 et est conduite vers un récipient adapté ;
- retour au début du cycle pour la source suivante.

Cette configuration permet de traiter un grand nombre de sources rapidement. Ceci répond à un besoin industriel. La production mondiale annuelle de sources est, en effet, supérieure à 10 millions d'unités par an. Ces sources sont produites industriellement par des machines automatiques du type de celle divulguée dans le document [7]. L'expérience montre que la qualité de la production n'est pas uniforme. La fabrication de ces sources est délicate du fait des dimensions réduites et il est fréquent de constater une forte disparité dans les lots produits : certaines sources sont trop actives, d'autres ne le sont pas du tout, il existe des disparités dimensionnelles ou des problèmes de non-uniformité.

A l'aide du dispositif présenté, il est possible de fabriquer un détecteur multi-capteurs permettant de caractériser complètement les sources produites. Cet instrument serait de taille modeste (ordre de grandeur : cylindre de hauteur 30 cm pour un diamètre externe de 30 cm), le dispositif de maintien de la source se trouvant au centre. Tout autour de la source on peut placer différents instruments comme un spectromètre 7, une caméra vidéo 8, un dosimètre voire éventuellement un tube à rayons X miniature et son capteur associé (ces derniers éléments ne sont pas représentés sur la figure 1). Il faut souligner que ce système multi-capteurs est uniquement rendu possible par l'utilisation du dispositif de maintien de la source présenté ici.

Le spectromètre 7 a pour but de caractériser le spectre d'émission des sources (vérification de l'activité, de la présence des marqueurs, de la nature des polluants éventuels). En utilisant un collimateur adapté et en déplaçant la source 2 verticalement, on peut également mesurer la répartition de la radioactivité au sein de la source. Des spectromètres compacts ne nécessitant pas de refroidissement à l'azote liquide existent aujourd'hui sur le marché. Ils conviennent pour cette application.

La caméra vidéo 8 permet de visualiser la source 2. Avec un objectif adapté, on peut atteindre un grossissement de 20 à 50 sans difficulté. Ceci permet de s'assurer de l'état de surface de la source (qualité des soudures, apparence de la gaine...). A l'aide d'un logiciel de traitement d'image on peut également effectuer un contrôle dimensionnel. Grâce à ces mesures on peut également, connaissant le débit de gaz injecté, les dimensions et la position de la source, déduire la masse de cette dernière avec une bonne sensibilité. Des caméras miniatures adaptées existent sur le marché.

Un dosimètre permet de s'assurer du débit de dose émis. Il ne s'agit pas d'un capteur absolu mais simplement d'un instrument de transfert (nécessitant donc un étalonnage).

Enfin, grâce au tube à rayons X et à un capteur adapté, on peut réaliser une image de l'intérieur de la source. Cette image est utile pour juger de la qualité de la fabrication (épaisseur de la gaine, position des marqueurs...). Ceci peut également être réalisé par auto-radiographie, la source radioactive elle même se substituant au tube à rayons X.

Finalement, on peut adjoindre à l'ensemble un système de tri des sources selon les résultats obtenus par les différents capteurs. Les sources non conformes peuvent être ainsi écartées et les sources peuvent être triées en fonction du débit de dose émis. On estime qu'une source pourrait être complètement caractérisée en une dizaine de secondes, ceci correspond à 3 600 sources mesurées par jour (c'est-à-dire 10 heures) ou un million de sources par an (c'est-à-dire 300 jours).

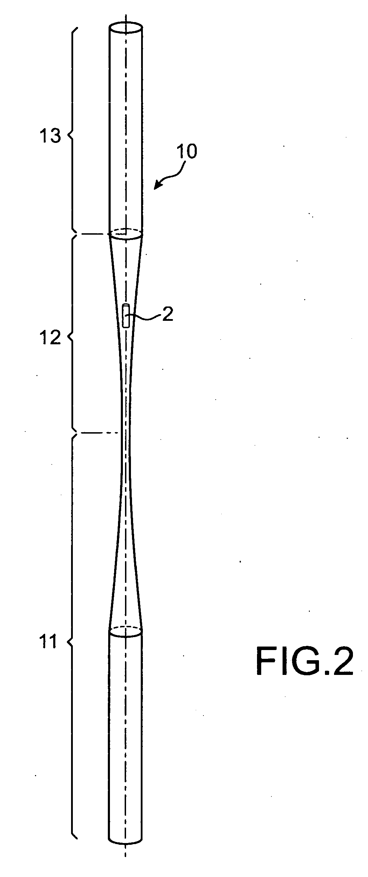

La figure 2 représente un autre tube utilisable par la présente invention. Le tube 10 comprend une partie inférieure 11, une partie médiane 12 et une partie supérieure 13. La partie inférieure 11 possède, avant sa jonction avec la partie médiane 12, un diamètre décroissant. La partie 12 possède, de sa jonction avec la partie inférieure 11 à sa jonction avec la partie supérieure 13, un diamètre croissant. La partie supérieure 13 possède un diamètre constant. On a représenté la source 2 à la hauteur désirée pour la caractériser.

Les premiers clients de ce genre d'instrument pourraient être les fabricants de sources (une vingtaine dans le monde) souhaitant caractériser la qualité de leur production et optimiser leur procédé de fabrication.

Une version simplifiée (moins de types de capteurs) de ce genre d'instrument pourrait également être développée. Cet instrument pourrait intéresser les praticiens et concerner de ce fait un marché beaucoup plus vaste. En effet, un appareil simple ne comportant que le seul spectromètre ou le dosimètre permettrait de caractériser 100% des sources avant implantation et ce en très peu de temps (de 20 à 30 minutes environ). Ceci constituerait un apport indéniable pour la qualité des soins. Il pourrait également être envisagé d'adapter le système présenté ici comme insert porte-source pour une chambre d'ionisation à puits conventionnelle. Moyennant une adaptation simple de l'instrumentation associée, le système proposé permettrait également d'automatiser les mesures dosimétriques d'un lot complet d'implants prostatiques. Il est également intéressant de souligner que l'appareil présenté dans la demande de brevet US 2004/0034268 pourrait aussi être amélioré (certainement simplifié) par l'utilisation du dispositif décrit dans la présente invention.

### Documents cités dans la description

[1] brevet US 5 095 217
[2] « New National Air-Kerma-Strength Standards for 125I and 103Pd Brachytherapy Seeds », S. M. Seltzer et al., J. Res. Natl. Inst. Stand. Technol. 108, 337-358 (2003).
[3] « Large-volume ionization chamber with variable apertures for air-kerma measurements of low-energy radiation sources », W. S. culberson et al., Rev. Sci, Instrum. 77, 015105 (2006).
[4] WO-A-01/89631
[5] demande de brevet US 2004/0034268
[6] brevet US 6 030 013
[7] demande de brevet US 2002/0162828

## Revendications

1. Système de maintien et de positionnement d'au moins un élément miniaturisé (2) se présentant sous la forme d'un cylindre, le système comprenant un tube (1) avec une partie supérieure (13), une partie médiane (12) et une partie inférieure (11), la partie supérieure (13) étant pourvue d'un orifice d'introduction de l'élément et permettant le déplacement de l'élément (2) jusqu'à la partie médiane (12) qui présente un diamètre légèrement supérieur au diamètre du cylindre formant l'élément (2), la partie inférieure (11) étant raccordée à des moyens (3) d'injection contrôlée d'un gaz pour injecter un gaz à l'intérieur du tube (1) de manière à mettre l'élément (2) en lévitation à une hauteur désirée dans la partie médiane (12) du tube, **caractérisé en ce que** la partie inférieure (11) du tube est pourvue d'un orifice d'évacuation de l'élément, et **en ce que** le tube comprend également des moyens (5) d'élimination de l'électricité statique présente à l'intérieur du tube (1).

2. Système selon la revendication 1, **caractérisé en ce que** la partie supérieure (13) du tube (1) est d'un diamètre supérieur au diamètre de la partie médiane (12) du tube (1) et est reliée à la partie médiane (12) par une partie en forme d'entonnoir.

3. Système selon l'une des revendications 1 ou 2, **caractérisé en ce que** le diamètre de la partie médiane (12) du tube (1) est croissant en partant de la partie inférieure (11) du tube (1) jusqu'à arriver à la partie supérieure (13) du tube.

4. Système selon l'une quelconque des revendications 1 à 3, **caractérisé en ce que** les moyens (3) d'injection d'un gaz sont des moyens d'injection d'un gaz choisi parmi les gaz rares, l'air, l'azote et un mélange d'azote et d'oxygène.

5. Système selon l'une quelconque des revendications 1 à 4, **caractérisé en ce que** les moyens d'élimination de l'électricité statique comprennent un fil électriquement conducteur (5) disposé à l'intérieur du tube (1) et relié à la terre.

6. Système selon la revendication 5, **caractérisé en ce que** le fil (5) électriquement conducteur est en un matériau choisi parmi Al, Al/Si et la fibre de carbone.

7. Système selon l'une quelconque des revendications 1 à 4, **caractérisé en ce que** les moyens d'élimination de l'électricité statique comprennent des moyens d'apport à l'intérieur du tube (1) de charges électriques opposées à celles fournies par l'électricité statique.

8. Système selon la revendication 7, **caractérisé en ce que** les moyens d'apport de charges électriques comprennent un fil électriquement conducteur disposé à l'intérieur du tube (1) et porté à un potentiel électrique lui permettant de produire lesdites charges électriques.

9. Système selon l'une quelconque des revendications 1 à 8, **caractérisé en ce que** le tube (1) est en matériau transparent.

10. Système selon la revendication 9, **caractérisé en ce que** le tube (1) est en quartz.

11. Système selon l'une quelconque des revendications 1 à 10, **caractérisé en ce que** les moyens d'injection d'un gaz comprennent un débitmètre massique (3).

12. Système selon l'une quelconque des revendications 1 à 11, **caractérisé en ce qu'**il comprend un capteur de position de la source asservissant le débit de gaz injecté à l'intérieur du tube (1) de façon à positionner ledit élément à la hauteur désirée.

13. Système selon l'une quelconque des revendications 1 à 12, **caractérisé en ce que** l'élément miniaturisé (2) est une source radioactive.

14. Appareil de caractérisation d'au moins une source radioactive miniaturisée (2), **caractérisé en ce qu'**il comprend un système de maintien et de positionnement selon la revendication 13 et des moyens de caractérisation de la source radioactive (2).

15. Appareil selon la revendication 14, **caractérisé en ce que** les moyens de caractérisation comprennent au moins un dispositif choisi parmi un spectromètre (7), une caméra vidéo (8), un dosimètre et un tube à rayons X et un capteur associé.

16. Appareil de caractérisation de sources radioactives miniaturisées (2) selon l'une des revendications 14 ou 15, **caractérisé en ce qu'**il comprend des moyens pour injecter successivement les sources radioactives (2) dans le tube (1), ces moyens d'injection injectant chaque nouvelle source à caractériser après que la source injectée précédemment a été mise en lévitation, **caractérisée** et éjectée du tube (1).

17. Appareil selon la revendication 16, **caractérisé en ce qu'**il comprend en outre des moyens d'obturation de l'extrémité inférieure du tube (1), pour obturer le tube (1) pendant la mise en lévitation et la caractérisation de la source (2) introduite dans le tube (1), et pour ouvrir le tube (1) afin de récupérer la source **caractérisée** (2).

## Claims

1. Support and positioning system for at least one miniaturised element (2) in the form of a cylinder, the system comprising a tube (1) with an upper part (13), a median part (12) and a lower part (11), the upper part (13) being provided with an orifice into which the element is inserted and enabling displacement of the element (2) as far as the median part (12) that has a diameter slightly greater than the diameter of the cylinder forming the element (2), the lower part (11) being connected to means (3) for controlled injection of a gas to inject a gas inside the tube (1) so as to levitate the element (2) to the required height in the median part (12) of the tube, **characterised in that** the lower part (11) of the tube is provided with an element removal orifice, and **in that** the tube also comprises means (5) of dissipating static electricity inside the tube (1).

2. System according to claim 1, **characterised in that** the upper part (13) of the tube (1) has a larger diameter than the diameter of the median part (11) of the tube (1) and is connected to the median part (11) through a funnel-shaped part.

3. System according to one of claims 1 or 2, **characterised in that** the diameter of the median part (12) of the tube (1) increases from the lower part (11) of the tube (1) until reaching the upper part (13) of the tube.

4. System according to any one of claims 1 to 3, **characterised in that** the gas injection means (3) are chosen from among rare gases, air, nitrogen and a mix of nitrogen and oxygen injection means.

5. System according to any one of claims 1 to 4, **characterised in that** the means of eliminating static electricity comprise an electrically conducting wire (5) arranged inside the tube (1) and connected to the ground.

6. System according to claim 5, **characterised in that** the electrically conducting wire (5) is made from a material chosen from among Al, Al/Si and carbon fibre.

7. System according to any one of claims 1 to 4, **characterised in that** the means of eliminating static electricity comprise means of bringing electrical charges opposing the charges supplied by static electricity into the tube (1).

8. System according to claim 7, **characterised in that** the means of bringing electrical charges comprise an electrically conducting wire arranged inside the tube (1) and brought to an electrical potential by which it can produce said electrical charges.

9. System according to any one of claims 1 to 8, **characterised in that** the tube (1) is made of a transparent material.

10. System according to claim 9, **characterised in that** the tube (1) is made of quartz.

11. System according to any one of claims 1 to 10, **characterised in that** the gas injection means comprise a mass flowmeter (3).

12. System according to any one of claims 1 to 11, **characterised in that** it comprises a source position sensor slaving the gas flow injected inside the tube (1) so as to position the said element at the required height.

13. System according to any one of claims 1 to 12, **characterised in that** the miniaturised element (2) is a radioactive source.

14. Characterisation device for at least one miniaturised radioactive source (2), **characterised in that** it comprises a support and positioning system according to claim 13 and means of characterisation of the radioactive source (2).

15. Device according to claim 14, **characterised in that** the characterisation means comprise at least one device chosen from among a spectrometer (7), a video camera (8), a dosimeter, an X-ray tube and an associated sensor.

16. Characterisation device for miniaturised radioactive sources (2) according to one of claims 14 or 15, **characterised in that** it comprises means of injecting radioactive sources (2) successively into the tube (1), these injection means injecting each new source to be **characterised** after the previously injected source has been put in levitation, **characterised** and ejected from the tube (1).

17. Device according to claim 16, **characterised in that** it also comprises means of closing off the lower end of the tube to close the tube (1) during levitation and characterisation of the source (2) inserted into the tube (1) and to open the tube (1) to recover the **characterised** source (2).

## Patentansprüche

1. Halte- und Positionierungssystem wenigstens eines die Form eines Zylinders aufweisenden Miniaturelements (2), wobei das System eine Röhre (1) mit einem eine Einführungsöffnung für das Element aufweisenden oberen Teil (13), einem mittleren Teil (12) und einem unteren Teil (11) umfasst und die Verschiebung des Elements (2) bis in den mittleren Teil (12) ermöglicht, der einen Durchmesser hat, der etwas größer ist als der Durchmesser des das Element (2) bildenden Zylinders, wobei der untere Teil (11) mit Einrichtungen (3) für den kontrollierten Einlass eines Gases verbunden ist, um ein Gas so ins Innere der Röhre (1) strömen zu lassen, dass sich das Element (2) in einer gewünschten Höhe des mittleren Teils (12) der Röhre im Schwebezustand befindet,
**dadurch gekennzeichnet, dass** der untere Teil (11) der Röhre eine Austrittsöffnung für das Element aufweist,
und **dadurch**, dass die Röhre auch Einrichtungen (5) zur Eliminierung der im Innern der Röhre (1) vorhandenen statischen Elektrizität umfasst.

2. System nach Anspruch 1, **dadurch gekennzeichnet, dass** der obere Teil (13) der Röhre (1) einen größeren Durchmesser hat als der mittlere Teil (12) der Röhre (1) und mit dem mittleren Teil (12) durch einen trichterförmigen Abschnitt verbunden ist bzw. mit einem trichterförmigen Abschnitt des Teils (12) verbunden ist.

3. System nach einem der Ansprüche 1 oder 2, **dadurch gekennzeichnet, dass** der Durchmesser des mittleren Teils (12) der Röhre (1) ab dem unteren Teil (11) der Röhre (1) bis zum oberen Teil (13) der Röhre zunimmt.

4. System nach einem der Ansprüche 1 bis 3, **dadurch gekennzeichnet, dass** die Einrichtungen (3) zum Einlassen eines Gases Einrichtungen zum Einlassen eines unter den Edelgasen, der Luft, dem Stickstoff und einem Stickstoff-Sauerstoff-Gemisch ausgewählten Gases sind.

5. System nach einem der Ansprüche 1 bis 4, **dadurch gekennzeichnet, dass** die Einrichtungen zur Eliminierung der statischen Elektrizität einen im Innern der Röhre (1) vorgesehenen, elektrisch leitfähigen und mit Erde verbundenen Draht (5) umfassen.

6. System nach Anspruch 5, **dadurch gekennzeichnet, dass** der elektrisch leitfähige Draht (5) aus einem unter Al, Al/Si und der Carbonfaser ausgewählten Material ist.

7. System nach einem der Ansprüche 1 bis 4, **dadurch gekennzeichnet, dass** die Einrichtungen zur Eliminierung der statischen Elektrizität Einrichtungen zur Einspeisung von elektrischen Ladungen ins Innere der Röhre (1) umfassen, die denjenigen entgegengesetzt sind, welche die statische Elektrizität liefert.

8. System nach Anspruch 7, **dadurch gekennzeichnet, dass** die Einrichtungen zur Einspeisung von elektrischen Ladungen einen elektrisch leitfähigen Draht umfassen, der im Innern der Röhre (1) angeordnet ist und auf ein zur Erzeugung der genannten elektrischen Ladungen fähiges Potential gebracht wird.

9. System nach einem der Ansprüche 1 bis 8, **dadurch gekennzeichnet, dass** die Röhre (1) aus transparentem Material ist.

10. System nach Anspruch 9, **dadurch gekennzeichnet, dass** die Röhre (1) aus Quarz ist.

11. System nach einem der Ansprüche 1 bis 10, **dadurch gekennzeichnet, dass** die Einrichtungen zum Einlassen eines Gases einen Massendurchflussmesser (3) umfassen.

12. System nach einem der Ansprüche 1 bis 11, **dadurch gekennzeichnet, dass** es einen Quellenpositionssensor umfasst, zur Regelung der ins Innere der Röhre (1) eingelassenen Gasmenge, um das genannte Element in der gewünschten Höhe zu positionieren.

13. System nach einem der Ansprüche 1 bis 12, **dadurch gekennzeichnet, dass** das Miniaturelement (2) eine radioaktive Quelle ist.

14. Vorrichtung zur Charakterisierung von wenigstens einer radioaktiven Miniaturquelle (2), **dadurch gekennzeichnet, dass** sie ein Halte- und Positionierungssystem nach Anspruch 13 sowie Charakterisierungseinrichtungen der radioaktiven Quelle (2) umfasst.

15. Vorrichtung nach Anspruch 14, **dadurch gekennzeichnet, dass** die Charakterisierungseinrichtungen wenigstens eine unter einem Spektrometer (7), einer Videokamera (8), einem Dosimeter und einer Röntgenstrahlröhre und einem zugeordneten Sensor ausgewählte Vorrichtung umfasst.

16. Vorrichtung zur Charakterisierung von radioaktiven Miniaturquellen (2) nach einem der Ansprüche 14 oder 15, **dadurch gekennzeichnet, dass** sie Einrichtungen zur sukzessiven Einführung der radioaktiven Quellen (2) in die Röhre (1) umfasst, wobei diese Einführungseinrichtungen jede neue zu charakterisierende Quelle einführen, nachdem die vorhergehend eingeführte Quelle in den Schwebezustand versetzt, charakterisiert und aus der Röhre (1) entfernt worden ist.

17. Vorrichtung nach Anspruch 16, **dadurch gekennzeichnet, dass** sie außerdem Verschlusseinrichtungen des unteren Endes der Röhre (1) umfasst, um die Röhre (1) während des Schwebezustands und der Charakterisierung der in die Röhre (1) eingeführten Quelle (2) zu erschließen, und um die Röhre (1) zur Rückgewinnung der charakterisierten Quelle (2) zu öffnen.
